# EUROPÄISCHE PATENTSCHRIFT

(11) **EP 2 019 874 B1**
(45) Veröffentlichungstag und Bekanntmachung des Hinweises auf die Patenterteilung: **11.11.2009**
(21) Anmeldenummer: 07729020.3
(22) Anmeldetag: 11.05.2007
(51) Int. Cl.: C25B 3/12, C07D 233/54, C07D 235/22

(54) **PORÖSES METALLORGANISCHES GERÜSTMATERIAL BASIEREND AUF PYRROLEN UND PYRIDINONEN**
POROUS METAL ORGANIC FRAMEWORK BASED ON PYRROLES AND PYRIDINONES
MATÉRIAU DE SQUELETTE MÉTALLO-ORGANIQUE POREUX À BASE DE PYRROLES ET DE PYRIDINONES

(30) Priorität: 16.05.2006 EP 06114001
(43) Veröffentlichungstag der Anmeldung: 04.02.2009
(73) Patentinhaber: BASF SE, 67056 Ludwigshafen (DE)
(72) Erfinder: RICHTER, Ingo, 68723 Schwetzingen (DE); SCHUBERT, Markus, 67063 Ludwigshafen (DE); MÜLLER, Ulrich, 67435 Neustadt (DE)
(74) Vertreter: Isenbruck, Günter
(86) Internationale Anmeldenummer: PCT/EP2007/054568
(87) Internationale Veröffentlichungsnummer: WO 2007/131955

(56) Entgegenhaltungen:
- WO-A-2005/049892
- DATABASE CA [Online] CHEMICAL ABSTRACTS SERVICE, COLUMBUS, OHIO, US; BUKHTIAROV, A. V. ET AL: "Cathodic nitrogen-hydrogen bond cleavage. Electrochemical reduction of nitrogen-containing heterocyclic compounds" XP002447400 gefunden im STN Database accession no. 1991:31704 & ZHURNAL OBSHCHEI KHIMII , 60(8), 1875-81 CODEN: ZOKHA4; ISSN: 0044-460X, 1990,
- DATABASE CA [Online] CHEMICAL ABSTRACTS SERVICE, COLUMBUS, OHIO, US; BOGDASHEV, N. N. ET AL: "Study of the anodic behavior of cobalt, nickel, and copper in methanol solutions during electrochemical synthesis of N-metal-substituted azoles" XP002447401 gefunden im STN Database accession no. 1979:514431 & INGIBIROVANIE PASSIVIROVANIE MET. , 111-16. EDITOR(S): BAGDASAROV, K. N. PUBLISHER: ROSTOVSKII UNIV., ROSTOV-ON-DON, USSR. CODEN: 40QRAH, 1976,
- DATABASE CA [Online] CHEMICAL ABSTRACTS SERVICE, COLUMBUS, OHIO, US; BOGDASHEV, N. N. ET AL: "Electrochemical method of the synthesis of N- and O-metal-substituted compounds of the azole series" XP002447402 gefunden im STN Database accession no. 1975:66041 & NOVOSTI ELEKTROKHIM. ORG. SOEDIN., TEZISY DOKL. VSES. SOVESHCH. ELEKTROKHIM. ORG. SOEDIN., 8TH , MEETING DATE 1973, 149-50. EDITOR(S): FEOKTISTOV, L. G. PUBLISHER: "ZINATNE", RIGA, USSR. CODEN: 28TSAX, 1973,
- HUANG XIAO-CHUN ET AL: "Ligand-directed strategy for zeolite-type metal-organic frameworks: zinc(II) imidazolates with unusual zeolitic topologies." ANGEWANDTE CHEMIE (INTERNATIONAL ED. IN ENGLISH) 27 FEB 2006, Bd. 45, Nr. 10, 27. Februar 2006 (2006-02-27), Seiten 1557-1559, XP002447389 ISSN: 1433-7851 in der Anmeldung erwähnt
- NAVARRO JORGE A R ET AL: "H2, N2, CO, and CO2 sorption properties of a series of robust sodalite-type microporous coordination polymers." INORGANIC CHEMISTRY 20 MAR 2006, Bd. 45, Nr. 6, 20. März 2006 (2006-03-20), Seiten 2397-2399, XP002447390 ISSN: 0020-1669 in der Anmeldung erwähnt

## Beschreibung

Die vorliegende Erfindung betrifft Verfahren zur Herstellung eines porösen metallorganischen Gerüstmaterials, enthaltend mindestens eine an mindestens ein Metallion koordinativ gebundene organische Verbindung.

Kristalline poröse metallorganische Gerüstmaterialien, sogenannte "Metal Organic Frameworks" (MOF) mit definierten Poren beziehungsweise Porenverteilungen und großen spezifischen Oberflächen sind gerade in jüngster Zeit Ziel umfangreicher Forschungstätigkeiten geworden.

Die bekanntesten porösen metallorganischen Gerüstmaterialien werden durch ein Metallion gebildet, an das eine Di-, Tri- oder Polycarbonsäure koordinativ derart gebunden ist, dass sich ein Koordinationspolymer ergibt, welches Poren aufweist.

Für die Darstellung solcher carbonsäurebasierten porösen metallorganischen Gerüstmaterialien sind in der Literatur zahlreiche Methoden beschrieben worden.

So beschreibt beispielsweise die US-A 5,648,508 mikroporöse metallorganische Gerüstmaterialien, die unter milden Reaktionsbedingungen aus einem Metallion und einem Liganden in Anwesenheit einer Templatverbindung hergestellt werden.

Eine weitere Methode carbonsäurebasierter poröser metallorganischer Gerüstmaterialien beruht auf der anodischen Oxidation eines Metalls in Gegenwart einer Carbonsäure zur Ausbildung eines porösen metallorganischen Gerüstmaterials. WO-A 2005/049892 beschreibt solche carbonsäurebasierten metallorganischen Gerüstmaterialien.

Aufgrund des Vorhandenseins von Carbonsäurefunktionalitäten ist es möglich, vergleichsweise robuste poröse metallorganische Gerüstmaterialien darzustellen.

Weitaus schwieriger ist die Darstellung von porösen metallorganischen Gerüstmaterialien, bei der die organische Komponente keinerlei funktionelle Gruppen aufweist, die zur Gerüstbildung geeignet sind. Hierbei wird allein durch das organische Gerüst ein koordinativer Aufbau ermöglicht.

Eine solche organische Verbindung stellt beispielsweise Imidazol dar, welches besonders interessante Eigenschaften, insbesondere in Bezug auf dessen Adsorptionsvermögen aufweist.

X.-C. Huang et al., Angew. Chem. 118 (2006), 1587 - 1589 beschreiben die Herstellung von Zink-2-methylimidazolid und Zink-2-ethylimidazolid sowie eines metallorganischen Gerüstmaterials, das sowohl 2-Ethyl- als auch 2-Methylimidazolid enthält.

Hierbei wird über einen Zeitraum von mehreren Tagen das entsprechende Imidazol in Methanol einer Zinkhydroxidlösung in wässrigem Ammoniak ausgesetzt.

Weitere Beispiele für solche organischen Verbindungen sind Triazole und 2- bzw. 4-Hydroxypyrimidin.

A.M. Goforth et al., J. Solid State Chem. 178 (2005), 2511-2518, beschreiben die Herstellung von porösen metallorganischen Gerüstmaterialien, die aus Zink und Triazolen aufgebaut sind, wobei das Zink in Form eines Zinkfluorids eingesetzt wird.

J.A.R. Navarro et al., Inorg. Chem. 45 (2006), 2397-2399, beschreiben 2- und 4-Hydroxypyrimidin basierte metallorganische Gerüstmaterialien, die aus einer Komplexvorstufe erzeugt werden. Diese metallorganischen Gerüstmaterialien weisen interessante Eigenschaften in Bezug auf deren Adsorptionsvermögen gegenüber Wasserstoff, Stickstoff, Kohlenmonoxid und Kohlendioxid auf.

All diesen organischen Gerüsten ist gemein, dass diese zwei Ringstickstoffatome aufweisen, die befähigt sind, ein Metall derart zu koordinieren, dass eine poröse Gerüststruktur aufgebaut wird und jeweils ein Ringstickstoff deprotoniert werden kann, wodurch die positive Ladung des Metallions kompensiert wird.

Trotz der befriedigenden Ausbeuten und ermittelten spezifischen Oberflächen besteht ein Bedarf an verbesserten Verfahren zur Herstellung solcher metallorganischer Gerüstmaterialien, deren organischer Bestandteil auf einer wie oben beschriebenen organischen Verbindung basiert.

Eine Aufgabe der vorliegenden Erfindung liegt somit darin, ein Verfahren zur Herstellung solcher porösen metallorganischen Gerüstmaterialien bereitzustellen.

Die Aufgabe wird gelöst durch ein Verfahren zur Herstellung eines porösen metallorganischen Gerüstmaterials, enthaltend mindestens eine an mindestens ein Metallion koordinativ gebundene organische Verbindung, den Schritt enthaltend

Oxidation mindestens einer dem mindestens einen Metallion entsprechenden Metall enthaltenden Anode in einem Reaktionsmedium in Gegenwart der mindestens einen organischen Verbindung, wobei die mindestens eine organische Verbindung ein Ringsystem darstellt, das ausgewählt ist aus der Gruppe bestehend aus 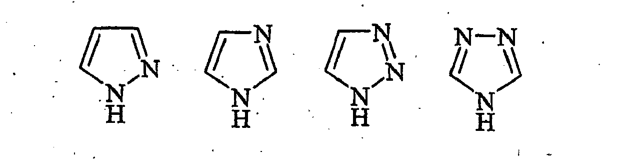 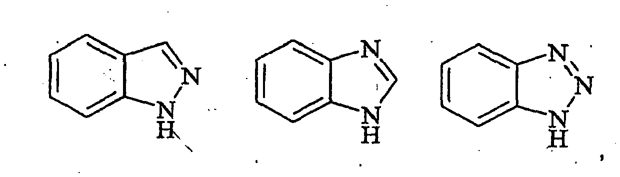 wobei das Ringsystem unsubstituiert ist oder einen oder mehrere Substituenten unabhängig ausgewählt aus der Gruppe bestehend aus Halogen, C₁₋₆ Alkyl, Phenyl, NH₂, NH(C₁₋₆ Alkyl), N(C₁₋₆ Alkyl)₂, OH, OPhenyl und OC₁₋₆ Alkyl aufweist, wobei die Substituenten C₁₋₆ Alkyl und Phenyl unsubstituiert sind oder einen oder mehrere Substituenten unabhängig ausgewählt aus der Gruppe bestehend aus Halogen, NH₂, NH(C₁₋₆ Alkyl), N(C₁₋₆ Alkyl)₂, OH, OPhenyl und OC₁₋₆ Alkyl aufweisen und wobei das Metall Zink ist.

Überraschenderweise wurde gefunden, dass durch die Bereitstellung des Metallions über anodische Oxidation des entsprechenden Metalls in Anwesenheit der mindestens einen organischen Verbindung ein entsprechendes poröses metallorganisches Gerüstmaterial gebildet werden kann, das im Vergleich zur im Stand der Technik bekannten Synthese eine höhere spezifische Oberfläche aufweist und mit höherer Ausbeute erhalten werden kann.

Bei dem erfindungsgemäßen Verfahren handelt es sich um die anodische Oxidation des mindestens einen Metalls, wobei dieses als Kation in das Reaktionsmedium eintritt und mit der mindestens einen organischen Verbindung zu einem porösen metallorganischen Gerüstmaterial reagiert. Dieses Gerüstmaterial kann beispielsweise durch Filtration abgetrennt werden.

Der Begriff "elektrochemische Herstellung", wie er im Rahmen der vorliegenden Erfindung verwendet wird, bezeichnet ein Herstellverfahren, bei dem in mindestens einem Verfahrensschritt die Bildung mindestens eines Reaktionsproduktes mit der Wanderung elektrischer Ladungen oder dem Auftreten von elektrischen Potentialen verbunden ist.

Der Begriff "mindestens ein Metallion", wie er im Rahmen der vorliegenden Erfindung verwendet wird, bezeichnet Ausführungsformen, gemäß denen mindestens ein Ion eines Metalls oder mindestens ein Ion eines ersten Metalls und mindestens ein Ion mindestens eines vom ersten Metall verschiedenen zweiten Metalls durch anodische Oxidation bereit gestellt werden.

Die vorliegende Erfindung umfasst auch Ausführungsformen, in denen mindestens ein Ion mindestens eines Metalls durch anodische Oxidation und mindestens ein Ion mindestens eines Metalls über ein Metallsalz bereit gestellt werden, wobei das mindestens eine Metall im Metallsalz und das mindestens eine Metall, das über anodische Oxidation als Metallion bereit gestellt werden, gleich oder voneinander verschieden sein können. Daher umfasst die vorliegende Erfindung beispielsweise eine Ausführungsform, gemäß der das Reaktionsmedium ein oder mehrere unterschiedliche Salze eines Metalls enthält und das in diesem Salz oder in diesen Salzen enthaltene Metallion zusätzlich durch anodische Oxidation mindestens einer dieses Metall enthaltenden Anode bereitgestellt wird. Ebenso umfasst die vorliegende Erfindung eine Ausführungsform, gemäß der das Reaktionsmedium ein oder mehrere unterschiedliche Salze mindestens eines Metalls enthält und mindestens ein von diesen Metallen unterschiedliches Metall über anodische Oxidation als Metallion im Reaktionsmedium bereitgestellt wird.

Gemäß einer bevorzugten Ausführungsform der vorliegenden Erfindung wird das mindestens eine Metallion durch anodische Oxidation mindestens einer der dieses mindestens eine Metall enthaltenden Anode bereitgestellt, wobei kein weiteres Metall über ein Metallsalz bereitgestellt wird.

In einer weiteren bevorzugten Ausführungsform enthält das nach dem erfindungsgemäßen Verfahren hergestellte metallorganische Gerüstmaterial nur ein Metall.

Demgemäß umfasst die vorliegende Erfindung eine Ausführungsform, gemäß der die mindestens eine Anode ein einziges oder zwei oder mehr Metalle enthält, wobei im Falle, dass die Anode ein einziges Metall enthält, dieses Metall durch anodische Oxidation bereitgestellt wird und im Falle, dass die Anode zwei oder mehr Metalle enthält, mindestens eines dieser Metalle durch anodische Oxidation bereitgestellt wird.

Weiterhin umfasst die vorliegende Erfindung eine Ausführungsform, gemäß der mindestens zwei Anoden verwendet werden, wobei die beiden gleich oder verschieden voneinander sein können. Jede der mindestens zwei Anoden kann hierbei ein einziges oder zwei oder mehr Metalle enthalten. Hierbei ist es beispielsweise möglich, dass zwei unterschiedliche Anoden die gleichen Metalle, diese jedoch in unterschiedlichen Anteilen enthalten. Ebenso ist es beispielsweise im Falle unterschiedlicher Anoden möglich, dass eine erste Anode ein erstes Metall enthält und eine zweite Anode ein zweites Metall enthält, wobei die erste Anode das zweite Metall und/oder die zweite Anode das erste Metall nicht enthält.

Das Metall ist Zink.

Als Metallion, das über anodische Oxidation im Reaktionsmedium bereit gestellt wird, Zn²⁺ zu nennen.

Der Aufbau der im erfindungsgemäßen Verfahren eingesetzten Anode kann grundsätzlich beliebig gewählt werden, solange gewährleistet ist, dass durch anodische Oxidation das mindestens eine Metallion im Reaktionsmedium zur Bildung des porösen metallorganischen Gerüstmaterials bereitgestellt werden kann.

Unter anderem bevorzugt sind Anoden in Form eines Stabs und/oder eines Rings und/oder einer Scheibe wie beispielsweise einer Ringscheibe und/oder einer Platte und/oder eines Rohrs und/oder einer Schüttung und/oder eines Zylinders und/oder eines Kegels und/oder eines Kegelstumpfs.

Gemäß einer bevorzugten Ausführungsform wird das erfindungsgemäße Verfahren unter Verwendung mindestens einer Opferanode durchgeführt. Der Begriff "Opferanode", wie er im Rahmen der vorliegenden Erfindung verwendet wird, bezeichnet eine Anode, die sich im Laufe des erfindungsgemäßen Verfahrens mindestens teilweise auflöst. Dabei werden auch Ausführungsformen erfasst, bei denen mindestens ein Teil des sich aufgelösten Anodenmaterials im Lauf des Verfahrens ersetzt wird. Dies kann beispielsweise dadurch erfolgen, dass mindestens eine neue Anode in das Reaktionssystem eingebracht wird oder gemäß einer bevorzugten Ausführungsform eine Anode in das Reaktionssystem eingebracht wird und im Verlauf des erfindungsgemäßen Verfahrens kontinuierlich oder diskontinuierlich in das Reaktionssystem nachgeführt wird.

Bevorzugt werden im erfindungsgemäßen Verfahren Anoden eingesetzt, die aus dem mindestens einen Metall, das als Metallionenquelle dient, bestehen oder dieses mindestens eine Metall auf mindestens ein geeignetes Trägermaterial aufgebracht enthalten.

Die Geometrie des mindestens einen Trägermaterials unterliegt im Wesentlichen keinen Beschränkungen. Möglich ist beispielsweise der Einsatz von Trägermaterialien in Form eines Gewebes und/oder einer Folie und/oder eines Filzes und/oder eines Siebes und/oder eines Stabs und/oder einer Kerze und/oder eines Kegels und/oder eines Kegelstumpfes und/oder eines Rings und/oder einer Scheibe und/oder einer Platte und/oder eines Rohrs und/oder einer Schüttung und/oder eines Zylinders.

Als Trägermaterialien kommen erfindungsgemäß beispielsweise Metalle wie beispielsweise mindestens eines der oben genannten Metalle, Legierungen wie beispielsweise Stähle oder Bronzen oder Messing, Graphit, Filz oder Schäume in Betracht.

Ganz besonders bevorzugt sind Anoden, die aus dem mindestens einen Metall, das als Metallionenquelle dient, bestehen.

Der Aufbau der im erfindungsgemäßen Verfahren eingesetzten Kathod kann grundsätzlich beliebig gewählt werden, solange gewährleistet ist, dass durch anodische Oxidation das mindestens eine Metallion im Reaktionsmedium bereitgestellt werden kann.

Gemäß einer bevorzugten Ausführungsform des erfindungsgemäßen Verfahrens wird das elektrisch leitende Elektrodenmaterial der mindestens einen Kathode so gewählt, dass im Reaktionsmedium keine störende Nebenreaktion stattfindet. Als bevorzugte Kathodenmaterialien sind unter anderem Graphit, Kupfer, Zink, Zinn, Mangan, Eisen, Silber, Gold, Platin oder Legierungen wie beispielsweise Stähle, Bronzen oder Messing zu nennen.

Als unter anderem bevorzugte Kombinationen des als Metallionenquelle dienenden Anodenmaterials und des elektrisch leitenden Kathodenmaterials sind beispielsweise zu nennen:

| **Anode** | **Kathode** |
|---|---|
| Zink | Zink |
| Zink | Stahl |
| Zink | Eisen |

Die Geometrie der mindestens einen Kathode unterliegt im Wesentlichen keinen Beschränkungen. Möglich ist beispielsweise der Einsatz von Kathoden in Form eines Stabs und/oder eines Rings und/oder einer Scheibe und/oder einer Platte und/oder eines Rohrs.

Im Rahmen der vorliegenden Erfindung kam im Wesentlichen jeder der in der Elektrochemie üblichen Zellentypen verwendet werden. Ganz besonders bevorzugt ist im erfindungsgemäßen Verfahren eine Elektrolysezelle, die für den Einsatz von Opferelektroden geeignet ist.

Grundsätzlich ist es unter anderem möglich, geteilte Zellen mit beispielsweise planparalleler Elektrodenanordnung oder kerzenförmigen Elektroden einzusetzen. Als Trennmedium zwischen den Zellkompartimenten können beispielsweise lonenaustauschermembranen, mikroporöse Membranen, Diaphragmen, Filtergewebe aus nicht-elektronenleitenden Materialien, Glasfritten und/oder poröse Keramiken eingesetzt werden. Vorzugsweise werden lonenaustauschermembranen, insbesondere Kationenaustauschermembranen, verwendet, wobei darunter wiederum solche Membranen vorzugsweise verwendet werden, die aus einem Copolymer aus Tetrafluorethylen und einem perfluorierten Monomer, das Sulfonsäuregruppen enthält, bestehen.

Im Rahmen einer bevorzugten Ausführungsform des erfindungsgemäßen Verfahrens werden bevorzugt eine oder mehrere ungeteilte Zellen eingesetzt.

Demgemäß betrifft die vorliegende Erfindung auch ein wie oben beschriebenes Verfahren, wobei das Verfahren in einer ungeteilten Elektrolysezelle durchgeführt wird.

Ganz besonders bevorzugt werden Kombinationen von Geometrien aus Anode und Kathode, bei denen die einander zugewandten Seiten der Anode und Kathode gemeinsam einen Spalt von homogener Dicke ausbilden.

In der mindestens einen ungeteilten Zelle werden die Elektroden beispielsweise bevorzugt planparallel angeordnet, wobei der Elektrodenspalt eine homogene Dicke beispielsweise im Bereich von 0,5 mm bis 30 mm, bevorzugt im Bereich von 0,75 mm bis 20 mm und besonders bevorzugt im Bereich von 1 bis 10 mm aufweist.

Im Rahmen einer bevorzugten Ausführungsform ist es beispielsweise möglich, eine Kathode und eine Anode derart planparallel anzuordnen, dass in der entstehenden Zelle ein Elektrodenspalt mit einer homogenen Dicke im Bereich von 0,5 bis 30 mm, bevorzugt im Bereich von 1 bis 20 mm, weiter bevorzugt im Bereich von 5 bis 15 mm und insbesondere bevorzugt im Bereich von 8 bis 12 mm wie beispielsweise im Bereich von ungefähr 10 mm ausgebildet wird. Diese Art der Zelle wird im Rahmen der vorliegenden Erfindung mit dem Begriff "Spaltzelle" bezeichnet.

Gemäß einer bevorzugten Ausführungsform des erfindungsgemäßen Verfahrens wird die oben stehend beschriebene Zelle als bipolar geschaltete Zelle eingesetzt.

Neben der oben beschriebenen Zelle werden gemäß einer ebenfalls bevorzugten Ausführungsform im Rahmen des erfindungsgemäßen Verfahrens die Elektroden einzeln oder zu mehreren gestapelt angewendet. Im letzteren Fall handelt es sich um so genannte Stapelelektroden, die in der demgemäß so genannten Plattenstapelzelle bevorzugt seriell bipolar geschaltet werden. Insbesondere für die Ausübung des erfindungsgemäßen Verfahrens im industriellen Maßstab werden bevorzugt mindestens eine Topfzelle und insbesondere bevorzugt seriell geschaltete Plattenstapelzellen eingesetzt, deren prinzipieller Aufbau in der DE 195 33 773 A1 beschrieben ist.

Im Rahmen der bevorzugten Ausführungsform der Plattenstapelzelle ist es beispielsweise bevorzugt, Scheiben aus geeigneten Materialien wie beispielsweise Kupferscheiben derart planparallel anzuordnen, dass zwischen den einzelnen Scheiben jeweils ein Spalt mit einer homogenen Dicke im Bereich von 0,5 bis 30 mm, bevorzugt im Bereich von 0,6 bis 20 mm, weiter bevorzugt im Bereich von 0,7 bis 10 mm, weiter bevorzugt im Bereich von 0,8 bis 5 mm und insbesondere im Bereich von 0,9 bis 2 mm wie beispielsweise im Bereich von ungefähr 1 mm ausgebildet wird. Dabei können die Abstände zwischen den einzelnen Scheiben gleich oder verschieden sein, wobei gemäß einer besonders bevorzugten Ausführungsform die Abstände zwischen den Scheiben im Wesentlichen gleich sind. Gemäß einer weiteren Ausführungsform kann sich das Material einer Scheibe der Plattenstapelzelle von dem Material einer anderen Scheibe der Plattenstapelzelle unterscheiden. Beispielsweise kann eine Scheibe aus Graphit, eine andere Scheibe aus Kupfer gefertigt sein, wobei die Kupferscheibe als Anode und die Graphitscheibe als Kathode geschaltet ist.

Weiterhin ist es im Rahmen der vorliegenden Erfindung beispielsweise bevorzugt, so genannte "pencil sharpener"-Zellen zu verwenden, wie sie beispielsweise in J. Chaussard et al., J. Appl. Electrochem. 19 (1989) 345-348 beschrieben sind. Insbesondere bevorzugt werden im erfindungsgemäßen Verfahren Pencil-Sharpener-Elektroden mit stabförmigen, nachführbaren Elektroden eingesetzt.

Insbesondere betrifft demgemäß die vorliegende Erfindung auch ein wie oben beschriebenes Verfahren, wobei das Verfahren in einer Spaltzelle oder Plattenstapelzelle durchgeführt wird.

Zellen, bei denen der Elektrodenabstand im Bereich von kleiner oder gleich 1 mm liegt, werden als Kapillarspaltzellen bezeichnet.

Gemäß ebenfalls bevorzugter Ausführungsformen des erfindungsgemäßen Verfahrens können Elektrolysezellen mit beispielsweise porösen Elektroden aus Metallschüttungen oder mit beispielsweise porösen Elektroden aus Metallnetzen oder mit beispielsweise Elektroden sowohl aus Metallschüttungen als auch Metallnetzen eingesetzt werden.

Gemäß einer weiteren bevorzugten Ausführungsform werden im erfindungsgemäßen Verfahren Elektrolysezellen eingesetzt, die mindestens eine Opferanode mit einem runden scheibenförmigen Querschnitt und mindestens eine Kathode mit einem ringförmigen Querschnitt aufweisen, wobei besonders bevorzugt der Durchmesser der bevorzugt zylinderförmigen Anode kleiner ist als der innere Durchmesser der Kathode und die Anode derart in der Kathode angebracht ist, dass zwischen der Außenfläche des Zylindermantels der Anode und der Innenfläche der die Anode zumindest teilweise umgebenden Kathode ein Spalt homogener Dicke gebildet wird.

Im Rahmen der vorliegenden Erfindung ist es auch möglich, durch Umpolung die ursprüngliche Anode zur Kathode und die ursprüngliche Kathode zur Anode zu machen. Im Rahmen dieser Verfahrensvariante ist es beispielsweise möglich, bei entsprechender Wahl von Elektroden, die unterschiedliche Metalle enthalten, zuerst ein Metall über anodische Oxidation als Metallkation zur Verfügung zu stellen und in einem zweiten Schritt nach Umpolung ein weiteres Metall zur Verfügung zu stellen. Ebenso ist es möglich, die Umpolung über das Anlegen von Wechselstrom zu bewerkstelligen.

Grundsätzlich ist es möglich, das Verfahren in Batch-Fahrweise oder kontinuierlich oder im Mischbetrieb durchzuführen. Bevorzugt wird das Verfahren kontinuierlich, insbesondere in mindestens einer Durchflusszelle, durchgeführt.

Die Spannungen, die im erfindungsgemäßen Verfahren angewendet werden, können an das jeweilige mindestens eine Metall der mindestens einen Anode angepasst werden, das als Metallionenquelle für das poröse metallorganische Gerüstmaterial dient, und/oder an die Eigenschaften der zumindest ersten organischen Verbindung und/oder gegebenenfalls an die Eigenschaften des untenstehend beschriebenen mindestens einen Lösungsmittels und/oder gegebenenfalls an die Eigenschaften des untenstehend beschriebenen mindestens einen Leitsalzes und/oder an die Eigenschaften der untenstehend beschriebenen mindestens einen kathodischen Depolarisationsverbindung angepasst werden.

Im Allgemeinen liegen die Spannungen pro Elektrodenpaar im Bereich von 0,5 bis 100 V, bevorzugt im Bereich von 1 bis 40 V und besonders bevorzugt im Bereich von 1,5 bis 20 V. Beispielsweise bevorzugte Bereiche sind etwa 1,5 bis 10 V oder 10 bis 20 V oder 20 bis 25 V oder 10 bis 25 V oder 4 bis 20 V oder 4 bis 25 V. Dabei kann die Spannung im Laufe des erfindungsgemäßen Verfahrens konstant sein oder sich im Verlauf des Verfahrens kontinuierlich oder diskontinuierlich ändern.

Beispielsweise im Fall, dass Kupfer anodisch oxidiert wird, liegen die Spannungen im Allgemeinen im Bereich von 3 bis 20 V, bevorzugt im Bereich von 3,5 bis 15 V und besonders bevorzugt im Bereich von 4 bis 15 V.

Die Stromdichten, die im Rahmen der erfindungsgemäßen Herstellung des porösen organischen Gerüstmaterials auftreten, liegen im Allgemeinen im Bereich von 0,01 bis 1000 mA/cm², bevorzugt im Bereich von 0,1 bis 1000 mA/cm², weiter bevorzugt im Bereich von 0,2 bis 200 mA/cm², weiter bevorzugt im Bereich von 0,3 bis 100 mA/cm² und besonders bevorzugt im Bereich von 0,5 bis 50 mA/cm².

Das erfindungsgemäße Verfahren wird im Allgemeinen bei einer Temperatur im Bereich von 0°C bis zum Siedepunkt, bevorzugt im Bereich von 20°C bis zum Siedepunkt des jeweiligen Reaktionsmediums oder des verwendeten, mindestens einen Lösungsmittels bevorzugt unter Normaldruck durchgeführt. Ebenso ist es möglich, das Verfahren unter Druck durchzuführen, wobei Druck und Temperatur bevorzugt so gewählt werden, dass das Reaktionsmedium bevorzugt zumindest teilweise flüssig ist.

Im Allgemeinen wird das erfindungsgemäße Verfahren bei einem Druck im Bereich von 0,5 bis 50 bar, bevorzugt im Bereich von 1 bis 6 bar und insbesondere bevorzugt bei Normaldruck durchgeführt.

Je nach Art und Aggregatzustand der Bestandteile des Reaktionsmediums kann die erfindungsgemäße elektrochemische Herstellung des metallorganischen Gerüstmaterials grundsätzlich auch ohne zusätzliches Lösungsmittel durchgeführt werden. Dies ist beispielsweise insbesondere dann der Fall, wenn die mindestens eine organische Verbindung im Reaktionsmedium als Lösungsmittel fungiert.

Ebenso ist es ohne Einsatz eines Lösungsmittels grundsätzlich möglich, das erfindungsgemäße Verfahren beispielsweise in der Schmelze durchzuführen, wobei mindestens ein Bestandteil des Reaktionsmediums in geschmolzenem Zustand vorliegt.

Gemäß einer bevorzugten Ausführungsform der vorliegenden Erfindung enthält das Reaktionsmedium mindestens ein geeignetes Lösungsmittel zusätzlich zu der mindestens einen organischen Verbindung und gegebenenfalls zu dem mindestens einen Leitsalz und gegebenenfalls zu der mindestens einen kathodischen Depolarisationsverbindung. Dabei kann die chemische Natur und die Menge dieses mindestens einen Lösungsmittels an die mindestens eine organische Verbindung und/oder an das mindestens eine Leitsalz und/oder an die mindestens eine kathodische Depolarisationsverbindung und/oder an das mindestens eine Metallion angepasst werden.

Als Lösungsmittel sind grundsätzlich alle Lösungsmittel oder alle Lösungsmittelgemische denkbar, in denen sich die im erfindungsgemäßen Verfahren eingesetzten Edukte unter den gewählten Reaktionsbedingungen wie Druck und Temperatur zumindest teilweise lösen oder suspendieren lassen. Im Rahmen der vorliegenden Erfindung umfasst der Begriff "Lösungsmittel" auch Lösungsmittelgemische. Beispielsweise eingesetzte Lösungsmittel sind unter anderem
- Wasser;
- Alkohole mit 1, 2, 3 oder 4 Kohlenstoffatomen wie Methanol, Ethanol, n-Propanol, iso-Propanol, n-Butanol, iso-Butanol, tert-Butanol;
- Carbonsäuren mit 1, 2, 3 oder 4 Kohlenstoffatomen wie Ameisensäure, Essigsäure, Propionsäure oder Butansäure;
- Nitrile wie beispielsweise Acetonitril oder Cyanobenzol;
- Ketone wie beispielsweise Aceton;
- Mindestens einfach halogensubstituierte niedere Alkane wie beispielsweise Methylenchlorid oder 1,2-Dichlorethan;
- Säureamide wie beispielsweise Amide von niederen Carbonsäuren wie beispielsweise Carbonsäuren mit 1, 2, 3 oder 4 Kohlenstoffatomen wie Amide der Ameisensäure, Essigsäure, Propionsäure oder Butansäure wie beispielsweise Formamid, Dimethylformamid (DMF), Diethylformamid (DEF), t-Butylformamid, Acetamid, Dimethylacetamid, Diethylacetamid oder t-Butyl-Acetamid;
- Cyclische Ether wie beispielsweise Tetrahydrofuran oder Dioxan;
- N-Formylamide oder N-Acetylamide oder symmetrische oder unsymmetrische Harnstoffderivate primärer, sekundärer oder cyclischer Amine wie beispielsweise Ethylamin, Diethylamin, Piperidin oder Morpholin;
- Amine wie beispielsweise Ethanolamin, Triethylamin oder Etyhlendiamin;
- Dimethylsulfoxid;
- Pyridin;
- Trialkylphosphite und Phosphate;
oder Gemische aus zwei oder mehr der vorgenannten Verbindungen.

Bevorzugt enthält das Reaktionsmedium ein organisches Lösungsmittel, das gegebenenfalls in einer Mischung mit Wasser vorliegt, insbesondere bevorzugt enthält das organische Lösungsmittel einen Alkohol.

Unter dem Begriff "organisches Lösungsmittel", wie er obenstehend verwendet wird, fallen sowohl reine organische Lösungsmittel als auch organische Lösungsmittel, die in geringen Mengen mindestens eine weitere Verbindung wie beispielsweise bevorzugt Wasser enthalten. In diesem Fall liegen die Wassergehalte der oben genannten Lösungsmittel im Bereich von bis zu 1 Gew.-%, bevorzugt im Bereich von bis zu 0,5 Gew.-%, besonders bevorzugt im Bereich von 0,01 bis 0,5 Gew.-% und insbesondere bevorzugt im Bereich von 0,1 bis 0,5 Gew.-%. Unter dem Begriff "Methanol" oder "Ethanol" oder "Acetonitril" oder "DMF" oder "DEF" wird beispielsweise im Rahmen der vorliegenden Erfindung auch ein Lösungsmittel verstanden, das jeweils insbesondere bevorzugt Wasser im Bereich von 0,1 bis 0,5 Gew.-% enthalten kann. Die mindestens eine weitere Verbindung kann jedoch auch anderer chemischer Natur sein. Insbesondere muss es sich hierbei nicht um ein gängiges Lösungsmittel handeln. Beispielhaft seien Stabilisatoren zu nennen. Sofern eine Mischung aus organischen Lösungsmitteln mit Wasser vorliegt, können selbstverständlich höhere Anteile an Wasser im Lösungsmittelgemisch vorhanden sein.

Als bevorzugte Lösungsmittel werden im erfindungsgemäßen Verfahren Methanol, Ethanol, Acetonitril, DMF und DEF oder ein Gemisch aus zwei oder mehr dieser Verbindungen eingesetzt. Ganz besonders bevorzugt sind als Lösungsmittel Methanol, Ethanol, DMF, DEF und ein Gemisch aus zwei oder mehr dieser Verbindungen. Insbesondere ist Methanol bevorzugt.

Im Rahmen einer bevorzugten Ausführungsform wird als Lösungsmittel mindestens ein protisches Lösungsmittel eingesetzt. Dieses wird unter anderem dann bevorzugt eingesetzt, wenn zur Vermeidung der untenstehend beschriebenen Wiederabscheidung des durch anodische Oxidation bereitgestellten mindestens einen Metallions an der Kathode die kathodische Bildung von Wasserstoff erreicht werden soll.

Im Rahmen der vorliegenden Erfindung kann jedoch auch auf ein protisches Lösungsmittel verzichtet werden, da die mindestens eine organische Verbindung zumindest einen Ringstickstoff aufweist, an den - zumindest durch eine Grenzformel wiedergegeben - ein Wasserstoffatom gebunden ist, das abgespalten und reduziert werden kann.

Beispielsweise im Fall, dass Methanol als Lösungsmittel eingesetzt wird, liegt die Temperatur für das erfindungsgemäße Verfahren unter Normaldruck im Allgemeinen im Bereich von 0 bis 90°C; bevorzugt im Bereich von 0 bis 65°C und insbesondere bevorzugt im Bereich von 15 bis 65°C.

Beispielsweise im Fall, dass Ethanol als Lösungsmittel eingesetzt wird, liegt die Temperatur im erfindungsgemäßen Verfahren unter Normaldruck im Allgemeinen im Bereich von 0 bis 100°C; bevorzugt im Bereich von 0 bis 78°C und insbesondere bevorzugt im Bereich von 25 bis 78°C.

Der pH-Wert des Reaktionsmediums wird im erfindungsgemäßen Verfahren so eingestellt, dass er für die Synthese oder die Stabilität oder bevorzugt für die Synthese und die Stabilität des Gerüstmaterials günstig ist. Beispielsweise kann der pH-Wert über das mindestens eine Leitsalz eingestellt werden.

Wird die Reaktion als Batch-Reaktion durchgeführt, liegt die Reaktionsdauer im Allgemeinen im Bereich von bis zu 30 h, bevorzugt im Bereich von bis zu 20 h. weiter bevorzugt im Bereich von 1 bis 10 h und insbesondere bevorzugt im Bereich von 1 bis 5 h.

Bei der mindestens einen organischen Verbindung handelt es sich um ein Ringsystem, das ausgewählt ist aus der Gruppe bestehend aus 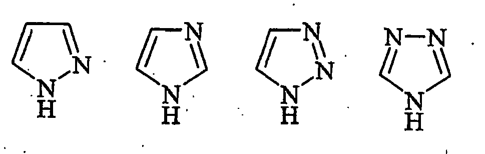 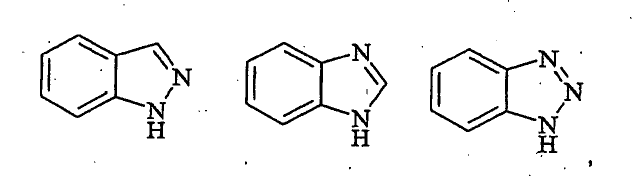 wobei das Ringsystem unsubstituiert ist oder einen oder mehrere Substituenten unabhängig ausgewählt aus der Gruppe bestehend aus Halogen, C₁₋₆ Alkyl, Phenyl, NH₂, NH(C₁₋₆ Alkyl), N(C₁₋₆ Alkyl)₂, OH, OPhenyl und OC₁₋₆ Alkyl aufweist, wobei die Substituenten C₁₋₆ Alkyl und Phenyl unsubstituiert sind oder einen oder mehrere Substituenten unabhängig ausgewählt aus der Gruppe bestehend aus Halogen, NH₂, NH(C₁₋₆ Alkyl), N(C₁₋₆ Alkyl)₂, OH, OPhenyl und OC₁₋₆ Alkyl aufweisen.

Der Begriff "C₁₋₆-Alkyl" bedeutet im Rahmen der vorliegenden Erfindung eine Alkylgruppe mit 1 bis 6 Kohlenstoffatomen. Beispiele hierfür sind Methyl, Ethyl, n-Propyl, i-Propyl, n-Butyl, i-Butyl, sec-Butyl, t-Butyl, Pentyl, Hexyl. Bevorzugte Reste sind Methyl und Ethyl. Sofern ein substituierter C₁₋₆-Alkylrest vorliegt, ist mindestens ein Wasserstoffatom durch einen anderen Substituenten ersetzt.

Im Rahmen der vorliegenden Erfindung bedeutet weiterhin der Begriff "Halogen" Fluor, Chlor, Brom oder Iod. Bevorzugt sind Fluor und Chlor.

Das Ringsystem kann unsubstituiert sein.

Darüber hinaus kann das Ringsystem einen oder mehrere Substituenten aufweisen. Sofern mehrere Substituenten vorhanden sind, können diese gleich oder verschieden sein.

Die Substituenten, welche am Ringsystem gebunden sind, können Halogen, C₁₋₆-Alkyl, Phenyl, NH₂, NH(C₁₋₆-Alkyl), N(C₁₋₆-Alkyl)₂, OH, OPhenyl oder OC₁₋₆-Alkyl sein.

Sofern zumindest einer der oben genannten Substituenten am Ringsystem ein C₁₋₆-Alkyl oder Phenyl ist, können diese ebenfalls unsubstituiert sein oder einen oder mehrere Substituenten aufweisen. Auch hier ist für den Fall, dass mehrere Substituenten vorhanden sind, es möglich, dass diese gleich oder verschieden sind. Diese sind ausgewählt aus der Gruppe bestehend aus Halogen, NH₂, NH(C₁₋₆-Alkyl), N(C₁₋₆-Alkyl), N(C₁₋₆-Alkyl)₂, OH, OPhenyl und OC₁₋₆-Alkyl.

Sofern die Gruppe C₁₋₆-Alkyl mehrfach auftritt, können diese Alkylgruppen gleich oder verschieden sein.

Es ist bevorzugt, dass die Substituenten, welche an das Ringsystem gebunden sind, keine weiteren Substituenten aufweisen.

Bevorzugte Substituenten, die an das Ringsystem gebunden sind, sind C₁₋₆-Alkyl, Phenyl, NH₂ und OH. Weiterhin besonders bevorzugt sind C₁₋₆-Alkyl und NH₂. Besonders bevorzugt ist C₁₋₆-Alkyl.

Weiterhin bevorzugt handelt es sich bei dem Ringsystem um ein Imidazol, Benzimidazol, oder Triazol,

Ganz besonders bevorzugt ist die mindestens eine organische Verbindung ausgewählt aus der Gruppe bestehend aus 2-Methylimidazol, 2-Ethylimidazol, Benzimidazol, 1,2,4-Triazol, 3-Amino-1,2,4-triazol, und 3,5-Diamino-1,2,4-triazol, beziehungsweise deren deprotonierte Form.

Es kann an der Gerüstbildung des porösen metallorganischen Gerüstmaterials eine wie oben beschriebene organische Verbindung eingesetzt werden. Ebenso können jedoch auch mehrere solcher organischer Verbindungen eingesetzt werden.

Vorzugsweise wird jedoch nur eine wie oben beschriebene organische Verbindung eingesetzt, die an der Gerüstbildung beteiligt ist.

Demzufolge ist eine bevorzugte Ausführungsform des erfindungsgemäßen Verfahrens zur Herstellung eines porösen metallorganischen Gerüstmaterials dadurch gegeben, dass das poröse metallorganische Gerüstmaterial nur eine wie oben beschriebene organische Verbindung enthält.

Die mindes.ens eine organische Verbindung wird in einer Konzentration eingesetzt, die im Allgemeinen im Bereich von 0,1 bis 30 Gew.-%, bevorzugt im Bereich von 0,5 bis 20 Gew.-% und besonders bevorzugt im Bereich von 2 bis 10 Gew.-%, jeweils bezogen auf das Gesamtgewicht des Reaktionssystems abzüglich des Gewichts der Anode und der Kathode, liegt. Demgemäß umfasst der Begriff "Konzentration" in diesem Fall sowohl die im Reaktionsmedium gelöste als auch beispielsweise die im Reaktionsmedium gegebenenfalls suspendierte Menge der mindestens einen organischen Verbindung.

Gemäß einer bevorzugten Ausführungsform des erfindungsgemäßen Verfahrens wird die mindestens eine organische Verbindung in Abhängigkeit des Fortgangs der Elektrolyse und insbesondere in Abhängigkeit von der Zersetzung der Anode beziehungsweise Freisetzung des mindestens einen Metallions und/oder in Abhängigkeit der Bildung des porösen metallorganischen Gerüstmaterials kontinuierlich und/oder diskontinuierlich zugesetzt.

Es können weitere organische Verbindungen dem Elektrolyten als Template zugegeben werden, deren Anwesenheit für die Ausbildung einer gewünschten Struktur vorteilhaft sind.

Gemäß einer insbesondere bevorzugten Ausführungsform des erfindungsgemäßen Verfahrens enthält das Reaktionsmedium mindestens ein geeignetes Leitsalz. In Abhängigkeit von der eingesetzten mindestens einen organischen Verbindung und/oder dem gegebenenfalls eingesetzten Lösungsmittel ist es im erfindungsgemäßen Verfahren auch möglich, die Herstellung des porösen metallorganischen Gerüstmaterials ohne zusätzliches Leitsalz durchzuführen.

Hinsichtlich der im erfindungsgemäßen Verfahren einsetzbaren Leitsalze existieren im Wesentlichen keine Beschränkungen. Bevorzugt werden beispielsweise Salze von Mineralsäuren, Sulfonsäuren, Phosphonsäuren, Boronsäuren, Alkoxysulfonsäuren oder Carbonsäuren oder von anderen aciden Verbindungen wie beispielsweise Sulfonsäureamiden oder Imiden eingesetzt.

Mögliche anionische Komponenten des mindestens einen Leitsalzes sind demgemäß unter anderem Sulfat, Monoalkylsulfat, wie Monomethylsulfat, Nitrat, Nitrit, Sulfit, Disulfit, Phosphat, Hydrogenphosphat, Dihydrogenphosphat, Diphosphat, Triphosphat, Phosphit, Chlorid, Chlorat, Bromid, Bromat, Iodid, Iodat, Carbonat oder Hydrogencarbonat.

Als Kationenkomponente der erfindungsgemäß einsetzbaren Leitsalze sind unter anderem Alkalimetallionen wie etwa Li⁺, Na⁺, K⁺ oder Rb⁺, Erdalkalimetallionen wie etwa Mg²⁺, Ca²⁺, Sr²⁺ oder Ba ²⁺, Ammoniumionen oder Phosphoniumionen zu nennen.

Bezüglich der Ammoniumionen sind quaternäre Ammoniumionen und protonierte Mono-, Di- und Triamine zu nennen.

Beispiele für erfindungsgemäß bevorzugt eingesetzte quaternäre Ammoniumionen im erfindungsgemäßen Verfahren sind unter anderem
- symmetrische Ammoniumionen wie etwa Tetraalkylammonium mit bevorzugt C₁-C₄-Alkyl, beispielsweise Methyl, Ethyl, n-Propyl, iso-Propyl, n-Butyl, iso-Butyl. tert-Butyl, wie Tetramethylammonium, Tetraethylammonium, Tetrapropylammonium, Tetrabutylammonium oder
- unsymmetrische Ammoniumionen wie etwa unsymmetrische Tetraalkylammonium mit bevorzugt C₁-C₄-Alkyl, beispielsweise Methyl, Ethyl, n-Propyl, iso-Propyl, n-Butyl, iso-Butyl. tert-Butyl, wie beispielsweise Methyltributylammonium oder
- Ammoniumionen mit mindestens einem Aryl wie beispielsweise Phenyl oder Naphthyl oder mindestes einem Alkaryl wie beispielsweise Benzyl oder mindestens einem Aralkyl und mindestens einem Alkyl, bevorzugt C₁-C₄-Alkyl, beispielsweise Methyl, Ethyl, n-Propyl, iso-Propyl, n-Butyl, iso-Butyl, tert-Butyl, wie etwa Aryltrialkyl wie etwa Benzyltrimethylammonium oder Benzyltriethylammonium.

Gemäß einer bevorzugten Ausführungsform wird im erfindungsgemäßen Verfahren als Leitsalz Natriummethylsulfat oder Tributylmethylammonium-methylsulfat eingesetzt.

Im Rahmen einer unter anderem bevorzugten Ausführungsform des erfindungsgemäßen Verfahrens ist es möglich, über die kationische und/oder anionische Komponente des mindestens einen Leitsalzes Verbindungen in das Reaktionsmedium einzubringen, die für den Aufbau des porösen metallorganischen Gerüstmaterials eingesetzt werden. Insbesondere kann im erfindungsgemäßen Verfahren mindestens eine organische Verbindung über mindestens ein Leitsalz eingebracht werden, die im resultierenden porösen metallorganischen Gerüstmaterial enthalten ist.

Im Rahmen einer Ausführungsform des erfindungsgemäßen Verfahrens ist es somit möglich, zusätzlich zu der mindestens einen Anode als Metallionenquelle das Metallion über die kationische Komponente des mindestens einen Leitsalzes in das Reaktionsmedium einzubringen. Ebenso ist es möglich, über die kationische Komponente des mindestens einen Leitsalzes mindestens ein Metallion in das Reaktionsmedium einzubringen, das von dem mindestens einen über anodische Oxidation eingebrachte Metallion verschieden ist, wobei sich diese Verschiedenheit auf die Wertigkeit des Kations und/oder die Art des Metalls beziehen kann.

Demgemäß beschreibt die vorliegende Erfindung auch ein wie oben beschriebenes Verfahren, wobei das mindestens eine Leitsalz ein Salz der mindestens einen organischen Verbindung enthält.

Die Konzentration des mindestens einen Leitsalzes liegt im Rahmen des erfindungsgemäßen Verfahrens im Allgemeinen im Bereich von 0,01 bis 10 Gew.-%, bevorzugt im Bereich von 0,05 bis 5 Gew.-% und insbesondere bevorzugt im Bereich von 0,1 bis 3 Gew.-%, jeweils bezogen auf die Summe der Gewichte sämtlicher im Reaktionsmedium vorhandener Leitsalze und weiter bezogen auf das Gesamtgewicht des Reaktionsmediums ohne Berücksichtigung der Anoden und Kathoden.

Wird das erfindungsgemäße Verfahren in Batch-Fahrweise durchgeführt, so wird im Allgemeinen zuerst das Reaktionsmedium mit den Edukten bereitgestellt, anschließend Strom angelegt und dann umgepumpt.

Wird das Verfahren kontinuierlich durchgeführt, so wird im Allgemeinen aus dem Reaktionsmedium ein Teilstrom ausgeschleust, das darin enthaltene poröse metallorganische Gerüstmaterial isoliert und die Mutterlauge (das verbleibende Reaktionsmedium) zurückgefahren.

Gemäß einer insbesondere bevorzugten Ausführungsform wird das erfindungsgemäße Verfahren so durchgeführt, dass die Wiederabscheidung des durch anodische Oxidation freigesetzten Metallions an der Kathode verhindert wird.
Diese Wiederabscheidung wird erfindungsgemäß beispielsweise bevorzugt dadurch verhindert, dass eine Kathode eingesetzt wird, die in einem gegebenen Reaktionsmedium eine geeignete Wasserstoffüberspannung aufweist. Solche Kathoden sind beispielsweise die bereits oben genannten Graphit-, Eisen-, Kupfer-, Zink-, Zinn-, Mangan-, Silber-, Gold-, Platin-Kathoden oder Kathoden, die Legierungen wie etwa Stähle, Bronzen oder Messing enthalten.

Die Wiederabscheidung wird erfindungsgemäß beispielsweise bevorzugt weiter dadurch verhindert, dass im Reaktionsmedium ein Elektrolyt eingesetzt wird, der die kathodische Bildung von Wasserstoff begünstigt. Diesbezüglich ist unter anderem ein Elektrolyt bevorzugt, der mindestens ein protisches Lösungsmittel enthält. Bevorzugte Beispiele für solche Lösungsmittel sind obenstehend aufgeführt. Besonders bevorzugt sind hierbei Alkohole, insbesondere bevorzugt Methanol und Ethanol.

Die Wiederabscheidung wird erfindungsgemäß beispielsweise bevorzugt weiter dadurch verhindert, dass im Reaktionsmedium mindestens eine Verbindung enthalten ist, die zu einer kathodischen Depolarisation führt. Unter einer Verbindung, die zu einer kathodischen Depolarisation führt, wird im Rahmen der vorliegenden Erfindung jede Verbindung verstanden, die unter gegebenen Reaktionsbedingungen an der Kathode reduziert wird.

Als kathodische Depolarisatoren sind unter anderem Verbindungen bevorzugt, die an der Kathode hydrodimerisiert werden. Beispielsweise besonders bevorzugt sind in diesem Zusammenhang Acrylnitril, Acrylsäureester und Maleinsäureester wie beispielsweise weiter bevorzugt Maleinsäuredimethylester.

Als kathodische Depolarisatoren sind weiter unter anderem Verbindungen bevorzugt, die mindestens eine Carbonylgruppe enthalten, die an der Kathode reduziert wird. Beispiele für solche Carbonylgruppen enthaltenden Verbindungen sind etwa Ketone wie beispielsweise Aceton.

Als kathodische Depolarisatoren sind unter anderem Verbindungen bevorzugt, die mindestens eine Stickstoff-Sauerstoff-Bindung, eine Stickstoff-Stickstoff-Bindung und/oder eine Stickstoff-Kohlenstoff-Bindung aufweisen, die an der Kathode reduziert werden. Beispiele für solche Verbindungen sind etwa Verbindungen mit einer Nitrogruppe, mit einer Azogruppe, mit einer Azoxygruppe, Oxime, Pyridine, Imine, Nitrile und/oder Cyanate.

Im Rahmen des erfindungsgemäßen Verfahrens ist es weiter möglich, mindestens zwei der genannten Maßnahmen zur Verhinderung der kathodischen Wiederabscheidung zu kombinieren. Beispielsweise ist es möglich, sowohl einen Elektrolyten einzusetzen, der die kathodische Bildung von Wasserstoff begünstigt als auch eine Elektrode mit einer geeigneten Wasserstoffüberspannung einzusetzen. Ebenso ist es möglich, sowohl einen Elektrolyten einzusetzen, der die kathodische Bildung von Wasserstoff begünstigt als auch mindestens eine Verbindung zuzusetzen, die zu einer kathodischen Depolarisation führt. Ebenso ist es möglich, sowohl mindestens eine Verbindung zuzusetzen, die zu einer kathodischen Depolarisation führt, als auch eine Kathode mit einer geeigneten Wasserstoffüberspannung einzusetzen. Weiter ist es möglich, sowohl einen Elektrolyten einzusetzen, der die kathodische Bildung von Wasserstoff begünstigt, als auch eine Elektrode mit einer geeigneten Wasserstoffüberspannung einzusetzen als auch mindestens eine Verbindung zuzusetzen, die zu einer kathodischen Depolarisation führt.

Demgemäß betrifft die vorliegende Erfindung auch ein wie oben beschriebenes Verfahren, wobei die kathodische Wiederabscheidung des mindestens einen Metallions durch mindestens eine der folgenden Maßnahmen zumindest teilweise verhindert wird:
(i) Verwendung eines Elektrolyten, der die kathodische Bildung von Wasserstoff begünstigt;
(ii) Zusatz mindestens einer Verbindung, die zu einer kathodischen Depolarisation führt;
(iii) Einsatz einer Kathode mit einer geeigneten Wasserstoffüberspannung.

Ebenso betrifft die vorliegende Erfindung daher auch ein wie oben beschriebenes Verfahren, wobei der Elektrolyt gemäß (i) mindestens ein protisches Lösungsmittel, insbesondere einen Alkohol, weiter bevorzugt Methanol und/oder Ethanol enthält.

Wie bereits ausgeführt wurde, sind diese Maßnahmen nicht zwingend erforderlich, da durch die mindestens eine organische Verbindung im Prinzip eine Wasserstoffabscheidung möglich und eine ausreichende Leitfähigkeit vorhanden sein kann.

Gemäß einer insbesondere bevorzugten Ausführungsform wird das erfindungsgemäße Verfahren in Kreisfahrweise betrieben. Unter diesem "Elektrolysekreis" wird im Rahmen der vorliegenden Erfindung jede Verfahrensführung verstanden, bei der zumindest ein Teil des sich in der Elektrolysezelle befindlichen Reaktionssystem aus der Elektrolysezelle ausgeschleust, gegebenenfalls mindestens einem Zwischenbehandlungsschritt wie beispielsweise mindestens einer Temperaturbehandlung oder Zusatz und/oder Abtrennung mindestens einer Komponenten des ausgeschleusten Stroms unterworfen und in die Elektrolysezelle zurückgeführt wird. Besonders bevorzugt wird ein solcher Elektrolysekreis im Rahmen der vorliegenden Erfindung in Kombination mit einer Plattenstapelzelle, einer Rohrzelle oder einer Pencil-Sharpener-Zelle durchgeführt.

Typischerweise liegt das poröse metallorganische Gerüstmaterial als Suspension vor. Das Gerüstmaterial kann von seiner Mutterlauge abgetrennt werden. Dieser Abtrennvorgang kann grundsätzlich gemäß sämtlicher geeigneter Verfahren erfolgen. Bevorzugt wird das Gerüstmaterial durch Fest-Flüssig-Trennung, Zentrifugation, Extraktion, Filtration, Membranfiltration, Crossflow-Filtration, Diafiltration, Ultrafiltration, Flokkulation unter Verwendung von Flokkulationshilfsmitteln wie beispielsweise nicht-ionische, kationische und/oder anionische Hilfsmittel, pH-Stift durch Zusatz von Additiven wie beispielsweise Salzen, Säuren oder Basen, Flotation, Sprühtrocknung, Sprühgranulation, oder Evaporation der Mutterlauge bei erhöhten Temperaturen und/oder im Vakuum und Aufkonzentration des Feststoffs abgetrennt.

Das von dem porösen metallorganischen Gerüstmaterial abgetrennte Reaktionsmedium (Mutterlauge) kann verworfen werden. Vorzugsweise wird dieses jedoch der Reaktion zurückgeführt, so dass diese erneut für die Oxidation eingesetzt wird.

Nach dem Abtrennen können sich mindestens ein zusätzlicher Waschschritt, mindestens ein zusätzlicher Trocknungsschritt und/oder mindestens ein zusätzlicher Calcinierungsschritt anschließen.

Schließt sich im erfindungsgemäßen Verfahren mindestens ein Waschschritt an, so wird bevorzugt mit mindestens einem bei der Synthese verwendeten Lösungsmittel gewaschen.

Schließt sich im erfindungsgemäßen Verfahren, gegebenenfalls nach mindestens einem Waschschritt, mindestens ein Trocknungsschritt an, so wird der Gerüstmaterial-Feststoff bei Temperaturen im Allgemeinen im Bereich von 20 bis 200°C, bevorzugt im Bereich von 40 bis 120°C und besonders bevorzugt im Bereich von 56 bis 60°C getrocknet.

Ebenfalls bevorzugt ist das Trocknen im Vakuum, wobei die Temperaturen im Allgemeinen so gewählt werden können, dass das mindestens eine Waschmittel zumindest teilweise, bevorzugt im Wesentlichen vollständig aus dem kristallinen porösen metall-organischen Gerüstmaterial entfernt wird und zugleich die Gerüststruktur nicht zerstört wird.

Die Trocknungszeit liegt im Allgemeinen im Bereich von 0,1 bis 15 h, bevorzugt im Bereich von 0,2 bis 5 h und insbesondere bevorzugt im Bereich von 0,5 bis 1 h.

An den gegebenenfalls mindestens einen Waschschritt und gegebenenfalls mindestens einen Trocknungsschritt kann sich mindestens ein Calcinierungsschritt anschließen, bei dem die Temperaturen bevorzugt so gewählt werden, dass die Struktur des Gerüstmaterials nicht zerstört wird.

Insbesondere durch Waschen und/oder Trocknen und/oder Calcinieren ist es beispielsweise möglich, mindestens eine Templatverbindung, die gegebenenfalls zur erfindungsgemäßen elektrochemischen Herstellung des Gerüstmaterials eingesetzt wurde, zumindest teilweise, bevorzugt im Wesentlichen quantitativ zu entfernen.

Typischerweise wird das erfindungsgemäße Verfahren zur Herstellung eines porösen metallorganischen Gerüstmaterials in Wasser als Lösungsmittel unter Zusatz einer weiteren Base durchgeführt. Durch die bevorzugte Verwendung des organischen Lösungsmittels ist es nicht erforderlich, eine solche Base einzusetzen. Nichts desto trotz kann das Lösungsmittel für das erfindungsgemäße Verfahren derart gewählt werden, dass dieses als solches basisch reagiert, was jedoch nicht zwingend für die Durchführung des erfindungsgemäßen Verfahrens sein muss. Zudem kann das organische Lösungsmittel in einem Gemisch mit Wasser vorliegen.

Ebenso kann eine Base eingesetzt werden. Bevorzugt ist jedoch, dass keine zusätzliche Base eingesetzt wird.

Ergänzend oder alternativ zu den oben genannten Calcinierungs- und/oder Waschschritten kann die Entfernung der mindestens einen organischen Verbindung (Ligand) aus den Poren des porösen metallorganischen Gerüstmaterials durch die Behandlung des gebildeten Gerüstmaterials mit einem weiteren Lösungsmittel erfolgen. Hierbei wird in einer Art "Extraktionsverfahren" der Ligand entfernt und gegebenenfalls im Gerüstmaterial durch ein Lösungsmittelmolekül ersetzt. Diese schonende Methode ist insbesondere geeignet, wenn es sich bei dem Liganden um eine hochsiedende Verbindung handelt.

Die Behandlung erfolgt vorzugsweise mindestens 30 Minuten und kann typischerweise bis zu 2 Tagen durchgeführt werden. Dies kann bei Raumtemperatur oder erhöhter Temperatur geschehen. Vorzugsweise erfolgt dies unter erhöhter Temperatur, beispielsweise bei mindestens 40°C, bevorzugt 60°C. Weiterhin bevorzugt erfolgt die Extraktion bei der Siedetemperatur des eingesetzten Lösungsmittels statt (unter Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen, eingesetzt werden.

Als geeignete Lösungsmittel können beispielsweise C₁₋₆-Alkanol, Dimethylsulfoxid (DMSO), N,N-Dimethylformamid (DMF), N,N-Diethylformamid (DEF), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon oder Mischungen davon verwendet werden.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogeniertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann beziehungsweise können. Beispiele hierfür sind Chloroform, Dichlormethan, Tetrachlormethan, Dichlorethan, Hexan, Heptan, Oktan sowie Gemische davon.

Bevorzugt sind Methanol, Ethanol, Propanol, Aceton, MEK und Mischungen davon.

Ein ganz besonders bevorzugtes Extraktionslösungsmittel ist Methanol.

Das verwendete Lösungsmittel ist vorzugsweise wasserfrei.

### Beispiel 1 Zn"(2-Methyl-Imidazolat)₂

Der Elektrolyt; bestehend aus 3,0 g 2-Methylimidazol, 4 g NaMeSO₄, 178,6 ml Methanol und 71,4 ml Wasser wurde in den Zellkreislauf eingefüllt. Eine Leitfähigkeit von 4,8 mS/cm wurde gemessen.

Der Zellkreislauf bestand aus einer Rohrzelle, einem Glaskühler und einer Kreislaufpumpe. Die Pumpe förderte den Elektrolyten bzw. die entstehende Suspension mit ca. 100 1/h im Kreis.

Die Rohrzelle bestand aus einem Edelstahlrohr (Länge: 10,2 cm, Innendurchmesser: 1,75 cm) als Kathode und einem Zinkstab als Anode (Länge: 10,2 cm, Durchmesser: 1,4 cm, Oberfläche: 45 cm²). Die Anordnung in der Elektrolysezelle stellte durch verschiedene luftdichte Dichtungen und Verschraubungen sicher, dass die Elektroden konzentrisch angeordnet sind und garantieren einen ringsum homogenen Spalt zwischen Kathode und Anode, durch den der auf 20°C thermostatisierte Elektrolyt gepumpt wird.

Bei einer Stromstärke von 0,5 A und einer Zellspannung von 1,5 bis 3,2 V wurde bis zu einem Stromeinsatz von 1 Faraday pro Mol 2-Methylimidazol für 1,9 Stunden gefahren (0,97 Ah). Während des Versuches wurde die Zelle mit einem Inertgasstrom gespült, um entstehenden Wasserstoff zu entfernen und eine Knallgasbildung auszuschließen.

Nach beendeter Elektrolyse wurde der Elektrolyt filtriert und 2 x mit 50 ml MeOH gewaschen. Das kristalline Produkt wurde bei 80°C bei 5 mbar getrocknet und es wurden 3,4 g Zn"(2-Methyl-Imidazolat)₂ erhalten (Ausbeute 81%). Die Oberfläche wurde bestimmt nach Langmuir gemäß DIN 66135 und lag bei 1746 m²/g.

### Beispiel 2 Zn"(2-Methyl-Imidazolat)₂

Der Elektrolyt, bestehend aus 76,1 g 2-Methylimidazol, 85,8 g Methyltributylammoniummethylsulfat (MTBS), 1810 g Methanol und 750,2 g Wasser wurde in den Zellkreislauf eingefüllt. Eine Leitfähigkeit von 4,8 mS/cm wurde gemessen.

Der Zellkreislauf bestand aus einer Rohrzelle, einem Glaskühler und einer Kreislaufpumpe. Die Pumpe förderte den Elektrolyten bzw. die entstehende Suspension mit ca. 600 1/h im Kreis.

Die Rohrzelle bestand aus einem Edelstahlrohr (Länge: 55 cm, Innendurchmesser: 5 cm) als Kathode und einem Zinkstab als Anode (Länge: 55 cm, Durchmesser: 1,94 cm, Oberfläche: 3,41 cm²). Die Anordnung in der Elektrolysezelle stellte durch verschiedene luftdichte Dichtungen und Verschraubungen sicher, dass die Elektroden konzentrisch angeordnet sind und garantieren einen ringsum homogenen Spalt zwischen Kathode und Anode, durch den der auf 29°C thermostatisierte Elektrolyt gepumpt wird.

Bei einer Stromstärke von 5,1 A und einer Zellspannung von 4,6 bis 5 V wurde bis zu einem Stromeinsatz von 1 Faraday pro Mol 2-Methylimidazol für 4,8 Stunden gefahren (24,6 Ah). Während des Versuches wurde die Zelle mit einem Inertgasstrom gespült, um entstehenden Wasserstoff zu entfernen und eine Knallgasbildung auszuschließen.

Nach beendeter Elektrolyse wurde der Elektrolyt filtriert mit 300 g MeOH gewaschen. Das Gewicht der Zinkanode verringerte sich um 29,0 g. Das kristalline Produkt wurde bei 80°C bei 1 mbar getrocknet und es wurden 100,9 g Zn"(2-Methyl-Imidazolat)₂ erhalten (Ausbeute 98%). Die Oberfläche wurde bestimmt nach Langmuir gemäß DIN 66135 und lag bei 1718 m²/g.

### Beispiel 3 Zn"(Benzimidazolat)₂

Der Elektrolyt, bestehend aus 4,3 g Benzimidazol, 1 g Methyltributylammoniummethylsulfat und 254,7 g Methanol wurde in den Zellkreislauf eingefüllt. Eine Leitfähigkeit von 0,5 mS/cm wurde gemessen.

Der Zellkreislauf bestand aus einer Rohrzelle, einem Glaskühler und einer Kreislaufpumpe. Die Pumpe förderte den Elektrolyten bzw. die entstehende Suspension mit ca. 200 I/h im Kreis.

Die Rohrzelle bestand aus einem Edelstahlrohr (Länge: 10,2 cm, Innendurchmesser: 1,75 cm) als Kathode und einem Zinkstab als Anode (Länge: 10,2 cm, Durchmesser: 1,4 cm, Oberfläche: 45 cm²). Die Anordnung in der Elektrolysezelle stellte durch verschiedene luftdichte Dichtungen und Verschraubungen sicher, dass die Elektroden konzentrisch angeordnet sind und garantieren einen ringsum homogenen Spalt zwischen Kathode und Anode, durch den der auf 30°C thermostatisierte Elektrolyt gepumpt wird.

Bei einer Stromstärke von 0,2 A und einer Zellspannung von 3,0 bis 7,9 V wurde bis zu einem Stromeinsatz von 1 Faraday pro Mol Benzimidazol für 4,8 Stunden gefahren (0,97 Ah). Während des Versuches wurde die Zelle mit einem Inertgasstrom gespült, um entstehenden Wasserstoff zu entfernen und eine Knallgasbildung auszuschließen.

Nach beendeter Elektrolyse wurde der Elektrolyt filtriert und mit MeOH gewaschen. Das kristalline Produkt wurde bei 50°C bei 4 mbar getrocknet und es wurden 9,5 g Zn"(Benzimidazolat)₂*7MeOH erhalten (Ausbeute 98%). Die Oberfläche wurde nach 16h bei 50°C im Hochvakuum (Masseverlust 39%) bestimmt nach Langmuir gemäß DIN 66135 und lag bei 465 m²/g.

## Patentansprüche

1. Verfahren zur Herstellung eines porösen metallorganischen Gerüstmaterials, enthaltend mindestens eine an mindestens ein Metallion koordinativ gebundene organische Verbindung, den Schritt enthaltend
Oxidation mindestens einer dem mindestens einen Metallion entsprechenden Metall enthaltenden Anode in einem Reaktionsmedium in Gegenwart der mindestens einen organischen Verbindung, wobei die mindestens eine organische Verbindung ein Ringsystem darstellt, das ausgewählt ist aus der Gruppe bestehend aus 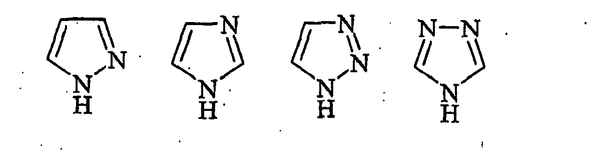 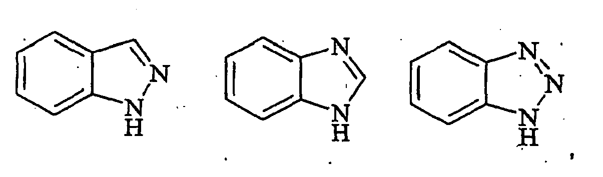 wobei das Ringsystem unsubstituiert ist oder einen oder mehrere Substituenten unabhängig ausgewählt aus der Gruppe bestehend aus Halogen, C₁₋₆ Alkyl, Phenyl, NH₂, NH(C₁₋₆ Alkyl), N(C₁₋₆ Alkyl)₂, OH, OPhenyl und OC₁₋₆ Alkyl aufweist, wobei die Substituenten C₁₋₆ Alkyl und Phenyl unsubstituiert sind oder einen oder mehrere Substituenten unabhängig ausgewählt aus der Gruppe bestehend aus Halogen, NH₂, NH(C₁₋₆ Alkyl), N(C₁₋₆ Alkyl)₂, OH, OPhenyl und OC₁₋₆ Alkyl aufweisen und wobei das Metall Zink ist.

2. Verfahren.nach Anspruch 1, **dadurch gekennzeichnet, dass** das poröse metallorganische Gerüstmaterial nur ein Metall enthält.

3. Verfahren nach Anspruch 1 oder 2, **dadurch gekennzeichnet, dass** das poröse metallorganische Gerüstmaterial nur eine organische Verbindung enthält.

4. Verfahren nach Anspruch 1 bis 3, **dadurch gekennzeichnet, dass** das Ringsystem ausgewählt ist aus der Gruppe bestehend aus Imidazol, Benzimidazol und Triazol.

5. Verfahren nach einem der Ansprüche 1 bis 4, **dadurch gekennzeichnet, dass** die mindestens eine organische Verbindung ausgewählt ist aus der Gruppe bestehend aus 2-Methylimidazol, 2-Ethylimidazol, Benzimidazol, 1,2,4-Triazol, 3-Amino-1,2,4-triazol und 3,5-Diamino-1,2,4-triazol beziehungsweise deren deprotonierte Form.

6. Verfahren nach einem der Ansprüche 1 bis 5, **dadurch gekennzeichnet, dass** das Reaktionsmedium ein organisches Lösungsmittel enthält.

7. Verfahren nach Anspruch 6, **dadurch gekennzeichnet, dass** das Lösungsmittel einen Alkohol enthält.

8. Verfahren nach einem der Ansprüche 1 bis 7, **dadurch gekennzeichnet, dass** die Oxidation in kontinuierlicher Fahrweise durchgeführt wird.

9. Verfahren nach einem der Ansprüche 1 bis 8, **dadurch gekennzeichnet, dass** das Reaktionsmedium nach Abtrennen des gebildeten porösen metallorganischen Gerüstmaterials erneut bei der Oxidation zur Herstellung des porösen metallorganischen Gerüstmaterials eingesetzt wird.

## Claims

1. A process for preparing a porous metal organic framework comprising at least one organic compound coordinated to at least one metal ion, which comprises the step
oxidation of at least one anode comprising metal corresponding to the at least one metal ion in a reaction medium in the presence of the at least one organic compound, where the at least one organic compound is a ring system which is selected from the group consisting of 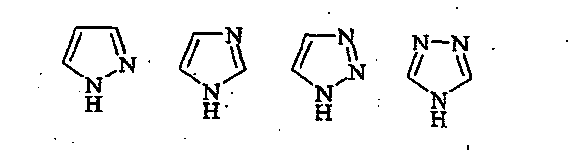 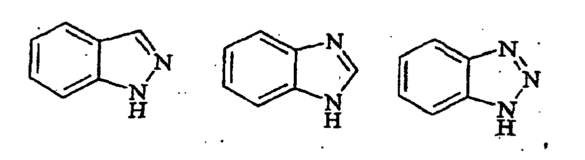 and is unsubstituted or bears one or more substituents selected independently from the group consisting of halogen, C₁₋₆-alkyl, phenyl, NH₂, NH(C₁₋₆-alkyl), N(C₁₋₆-alkyl)₂, OH, Ophenyl and OC₁₋₆-alkyl, where the substituents C₁₋₆-alkyl and phenyl are unsubstituted or bear one or more substituents selected independently from the group consisting of halogen, NH₂, NH(C₁₋₆-alkyl), N(C₁₋₆-alkyl)₂, OH, Ophenyl and OC₁₋₆-alkyl and where the metal is zinc.

2. The process according to claim 1, wherein the porous metal organic framework comprises only one metal.

3. The process according to claim 1 or 2, wherein the porous metal organic framework comprises only one organic compound.

4. The process according to claims 1 to 3, wherein the ring system is selected from the group consisting of imidazole, benzimidazole and triazole.

5. The process according to any of claims 1 to 4, wherein the at least one organic compound is selected from the group consisting of 2-methylimidazole, 2-ethylimidazole, benzimidazole, 1,2,4-triazole, 3-amino-1,2,4-triazole and 3,5-diamino-1,2,4-triazole and their deprotonated forms.

6. The process according to any of claims 1 to 5, wherein the reaction medium comprises an organic solvent.

7. The process according to claim 6, wherein the solvent comprises an alcohol.

8. The process according to any of claims 1 to 7, wherein the oxidation is carried out continuously.

9. The process according to any of claims 1 to 8, wherein the reaction medium after the porous metal organic framework formed has been separated off is reused in the oxidation for preparing the porous metal organic framework.

## Revendications

1. Procédé de fabrication d'un matériau de squelette métallo-organique poreux, contenant au moins un composé organique relié coordinativement à au moins un ion métallique, qui comprend l'étape
oxydation d'au moins une anode contenant un métal correspondant à l'ion métallique ou aux ions métalliques dans un milieu réactionnel en présence du ou des composés organiques, le ou les composés organiques étant des systèmes cycliques choisis dans le groupe constitué de 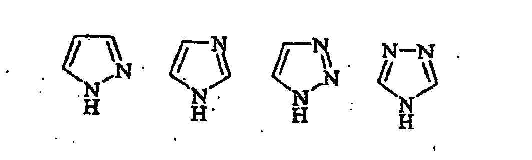 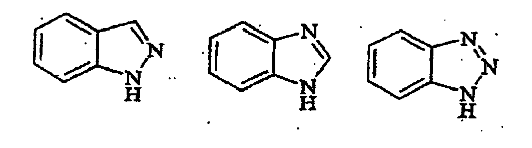 les systèmes cycliques étant non substitués ou comportant un ou plusieurs substituants choisis indépendamment dans le groupe constitué d'halogène, alkyle en C₁₋₆, phényle, NH₂, NH (alkyle en C₁₋₆), N (alkyle en C₁₋₆)₂, OH, O-phényle et O-alkyle en C₁₋₆, les substituants alkyle en C₁₋₆ et phényle étant non substitués ou comportant un ou plusieurs substituants choisis indépendamment dans le groupe constitué d'halogène, NH₂, NH(alkyle en C₁₋₆), N(alkyle en C₁₋₆)₂, OH, O-phényle et O-alkyle en C₁₋₆, et le métal étant le zinc.

2. Procédé selon la revendication 1, **caractérisé en ce que** le matériau de squelette métallo-organique poreux contient uniquement un métal.

3. Procédé selon la revendication 1 ou 2, **caractérisé en ce que** le matériau de squelette métallo-organique poreux contient uniquement un composé organique.

4. Procédé selon la revendication 1 à 3, **caractérisé en ce que** le système cyclique est choisi dans le groupe constitué de l'imidazol, du benzimidazol et du triazol.

5. Procédé selon l'une quelconque des revendications 1 à 4, **caractérisé en ce que** le ou les composés organiques sont choisis dans le groupe constitué du 2-méthylimidazol, du 2-éthylimidazol, du benzimidazol, du 1,2,4-triazol, du 3-amino-1,2,4-triazol et du 3,5-diamino-1,2,4-triazol ou leur forme déprotonée.

6. Procédé selon l'une quelconque des revendications 1 à 5, **caractérisé en ce que** le milieu réactionnel contient un solvant organique.

7. Procédé selon la revendication 6, **caractérisé en ce que** le solvant contient un alcool.

8. Procédé selon l'une quelconque des revendications 1 à 7, **caractérisé en ce que** l'oxydation est réalisée de manière continue.

9. Procédé selon l'une quelconque des revendications 1 à 8, **caractérisé en ce qu'**après la séparation du matériau de squelette métallo-organique poreux formé, le milieu réactionnel est de nouveau utilisé lors de l_{'}oxydation pour la fabrication du matériau de squelette métallo-organique poreux.
